# FASCICULE DE BREVET EUROPEEN

(11) **EP 4 108 282 B1**
(45) Date de publication et mention de la délivrance du brevet: **11.09.2024**
(21) Numéro de dépôt: 22172258.0
(22) Date de dépôt: 09.05.2022
(51) Int. Cl.: A61M 16/00, A61M 16/10, A61M 16/20, A61M 16/22

(54) **INSTALLATION DE FOURNITURE DE GAZ ADAPTÉE AU TRAITEMENT D'UN INDIVIDU AYANT BESOIN D'UN DÉBIT ÉLEVÉ D OXYGÈNE**
GASVERSORGUNGSANLAGE, DIE FÜR DIE BEHANDLUNG EINER PERSON GEEIGNET IST, DIE EINEN HOHEN SAUERSTOFFDURCHSATZ BENÖTIGT
INSTALLATION FOR PROVIDING GAS ADAPTED TO THE TREATMENT OF AN INDIVIDUAL NEEDING A HIGH FLOW OF OXYGEN

(30) Priorité: 24.06.2021 FR 2106772
(43) Date de publication de la demande: 28.12.2022
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, Newark, 19702 (US)
(74) Mandataire: Air Liquide

(56) Documents cités:
- EP-A1- 3 741 416
- EP-A2- 1 598 103
- WO-A1-98/22173
- US-A1- 2003 145 855
- US-A1- 2018 093 063

## Description

La présente invention concerne une installation de fourniture de gaz respiratoire à un individu, i.e. un être humain, typiquement un patient en état hypoxémique, comprenant une source d'oxygène (O₂) et un système d'épuration et de recyclage des gaz expirés riches en O₂ et CO₂, permettant d'obtenir un gaz purifié contenant de l'oxygène (O₂) à haute concentration, i.e. au moins 90% vol., et de l'additionner au flux d'oxygène provenant de la source d'oxygène. Une telle installation peut être utilisée pour traiter notamment une personne en état hypoxémique infectée par un coronavirus, telle Covid-19 ou analogue, que ce soit un adulte, notamment une personne âgée, un adolescent ou enfant.

L'administration d'oxygène (O₂) gazeux sert à corriger une situation hypoxémique, c'est-à-dire lorsque la saturation d'oxyhémoglobine dans le sang ou « saturation en oxygène » devient ou est inférieure à une valeur normale, e.g. hors de la plage 95-100%, en particulier inférieure à 90%, chez un individu humain, i.e. un patient dans une population de patients de type nouveaux nés, enfants ou adultes, souffrant d'une pathologie respiratoire, par exemple une Bronchopneumopathie Chronique Obstructive (BPCO) ou un Syndrome de Détresse Respiratoire Aigüe (SDRA), notamment dans le cadre de la pandémie lié au coronavirus Covid-19.

A l'hôpital, l'oxygène provient d'un (ou plusieurs) réservoir de grande capacité (e.g. stockage de plusieurs milliers de litres), notamment contenant de l'oxygène sous forme liquide (LOX), qui est d'abord vaporisé, puis acheminé et enfin délivré par une ou des prises de distribution de gaz murales agencées dans les pièces de l'hôpital ou analogue, notamment les chambres, les salles de soins.... Les prises sont connectées fluidiquement au réseau de canalisations d'oxygène de l'hôpital alimenté par le stockage de grande capacité. Une telle installation permet, en temps normal, d'assurer un apport continu d'O₂ gazeux aux patients. Le réservoir de grande capacité doit être régulièrement réapprovisionné en LOX, typiquement par camion-citerne.

Selon la criticité de l'état du patient, des débits d'oxygène pouvant être élevés, typiquement pouvant atteindre 15 L/min, voire plus. Le débit d'oxygène souhaité est généralement réglé au moyen d'un débitmètre raccordé fluidiquement à la prise murale fournissant l'oxygène, puis acheminé au patient en ayant besoin, via un conduit d'acheminement de gaz raccordé fluidiquement à la sortie du débitmètre et alimentant une interface respiratoire, tel un masque respiratoire naso-buccal (i.e. facial). Ceci permet d'obtenir une fraction inspirée en oxygène (FiO₂) par le patient qui est supérieure à 90% et, en général, proche de 100%.

Or, la pandémie liée au coronavirus COVID-19 a démultiplié le nombre de patients hypoxiques devant être traités avec de hautes concentrations d'O₂ pour rétablir leur saturation en oxygène normale, ce qui a engendré de nombreux problèmes d'approvisionnement en oxygène dans les pays développés et même rendu la tâche quasi-impossible dans les pays moins avancés, dans lesquels l'accès à l'O₂ est plus difficile, que ce soit pour des raisons de production, de logistique ou autre, entraînant une crise sanitaire inédite pour la population, en particulier pour les malades qui ont été privés ou limités en oxygène.

Par ailleurs, il est connu, notamment de US-A-4,859,217 et EP-A-785020, de pouvoir utiliser une installation d'adsorption par modulation de pression de type PSA (pour Pressure Swing Adsorption) pour séparer, directement sur site, l'air ambient et produire de l'O₂ gazeux en éliminant par adsorption sur tamis moléculaire, par exemple de la zéolite, au moins une partie des autres composés gazeux qui s'y trouvent, à savoir principalement de l'azote (N₂) et de l'argon (Ar). L'oxygène obtenu a une concentration de l'ordre de 90 à 95% en volume (reste Ar, N₂...). Une installation PSA, installée dans un bâtiment de soins, i.e. hôpital ou analogue, peut produire de l'oxygène gazeux à un débit pouvant atteindre environ 15 L/min d'O₂.

Toutefois, une telle installation PSA présente des inconvénients, notamment en termes d'encombrement, de poids, de coût et aussi de bruit du fait des phases de compression et décompression qu'elle met en oeuvre pour opérer les modulations de pression. Du fait de ces contraintes, ce type d'installation PSA est très peu utilisé en milieu hospitalier.

Afin de tenter de pallier ces inconvénients, EP-A-1157731 propose un concentrateur d'oxygène portable fonctionnant en mode PSA dont l'encombrement et le poids sont réduits. Si ce type de concentrateur permet de produire de l'oxygène gazeux à une pureté de l'ordre de 90 à 95 % vol., il ne permet pas de produire des quantités suffisantes d'oxygène, donc des débits suffisamment élevés pour garantir un traitement efficace des patients atteints de pathologies nécessitant des débits élevés d'oxygène à haute concentration, en particulier les personnes atteintes du coronavirus Covid-19 ou analogue.

Enfin, les dispositifs d'administration d'oxygène actuels alimentés par de telles installation de fourniture d'oxygène, conduisent à des pertes d'oxygène importantes car une proportion élevée d'oxygène ayant été inhalé par le patient, se retrouve dans les gaz riches en CO₂ expirés par le patient qui sont rejetés à l'atmosphère. En effet, tout l'oxygène présent dans le flux d'oxygène inhalé par le patient ne peut être assimilé par ses poumons et une partie non négligeable de cet oxygène, en général au moins 80 à 90%, est expirée et rejetée à l'atmosphère avec le CO₂ et la vapeur d'eau présente dans le gaz expiré, donc gaspillée.

Or, on comprend aisément que, lorsque l'oxygène disponible est en quantité restreinte, du fait notamment d'une demande importante, de problèmes de production, de logistique ou autre, ces pertes d'oxygène ne sont pas acceptables car tout l'oxygène perdu ne peut servir à soigner les patients.

US-A-2018/0093063 et US-A-2003/0145855 enseignent des systèmes de purification de gaz à deux adsorbeurs fonctionnant en alternance, et EP-A-3741416 décrit la purification de gaz expiré par le patient au moyen d'un lit unique d'adsorbant de type chaux sodée. Ces systèmes ne permettent pas d'assurer un fonctionnement en continu et une production suffisante d'oxygène purifié.

On connait aussi WO98/22173 qui enseigne une installation d'anesthésie ventilatoire ayant une architecture similaire au préambule de la revendication 1.

Au vu de cela, il convient de proposer une installation de fourniture de gaz, en particulier d'oxygène, améliorée de manière à pouvoir limiter la quantité d'oxygène utilisée et/ou perdue pour traiter un individu, i.e. un patient, dans le cadre d'un traitement par administration d'oxygène, c'est-à-dire en oxygénothérapie, notamment une personne hypoxique infectée par un virus, notamment un coronavirus, telle Covid-19, en particulier lorsque l'oxygène doit lui être fourni à haute concentration (e.g. >95% vol.).

Selon l'invention, il est proposé une installation de fourniture d'un gaz respiratoire à un utilisateur, tel un patient, comprenant :
- une source de gaz pour fournir un gaz respiratoire contenant de l'oxygène, c'est-à-dire un gaz riche en oxygène, tel de l'oxygène,
- une interface respiratoire pour administrer le gaz respiratoire à l'utilisateur, lors de chaque phase inspiratoire dudit utilisateur,
- une ligne principale d'acheminement de gaz reliant fluidiquement la source de gaz à l'interface respiratoire pour acheminer le gaz respiratoire de la source de gaz à l'interface respiratoire,
- une ligne de récupération de gaz en communication fluidique avec l'interface respiratoire pour récupérer et acheminer au moins une partie du mélange gazeux CO₂/O₂ expiré se retrouvant dans l'interface respiratoire lors de chaque phase expiratoire de l'utilisateur,
- un système de purification de gaz alimenté en mélange gazeux CO₂/O₂ expiré par la ligne de récupération de gaz, et configuré pour :
   ▪ éliminer au moins une partie du CO₂ contenu dans le mélange gazeux CO₂/O₂ expiré et
   ▪ obtenir un gaz purifié contenant majoritairement de l'O₂,
et une ligne de recyclage de gaz reliant fluidiquement le système de purification de gaz à la ligne principale d'acheminement de gaz pour acheminer au moins une partie du gaz purifié provenant du système de purification de gaz et le réinjecter dans la ligne principale d'acheminement de gaz.

De plus, le système de purification de gaz de l'installation selon l'invention comprend au moins trois, i.e. 3, adsorbeurs agencés en parallèle contenant chacun au moins un adsorbant, chaque adsorbeur étant configuré pour fonctionner selon des cycles d'adsorption/désorption comprenant :
▪ une phase d'adsorption pendant laquelle au moins une partie du CO₂ contenu dans le mélange gazeux CO₂/O₂ expiré est adsorbé par l'adsorbant, et
▪ une phase de régénération pendant laquelle au moins une partie du CO₂ adsorbé par l'adsorbant est désorbé et évacué à l'atmosphère.

L'installation de l'invention comprend en outre une turbine commandée par des moyens de pilotage à microprocesseur, ladite turbine étant agencée sur un conduit d'air relié fluidiquement à l'atmosphère et permettant de générer un débit d'air ambiant non-chauffé dans ledit conduit d'air, ledit débit d'air ambiant non-chauffé étant utilisé durant chaque phase de régénération pour régénérer au moins un adsorbant contenu dans au moins l'un des adsorbeurs en désorbant au moins une partie du CO₂ adsorbé par ledit adsorbant.

Une telle installation de fourniture de gaz constitue un ensemble de fourniture d'oxygène mettant en oeuvre une source d'oxygène gazeux permettant de délivrer un flux d'alimentation riche en oxygène gazeux, tel de l'oxygène pur (i.e. 100%vol.), d'une part, et un système d'épuration des gaz expirés, d'autre part, pour récupérer l'oxygène, normalement perdu, qui est présent dans les gaz expirés par le patient (en mélange avec du CO₂), puis le recycler ensuite dans le flux d'alimentation riche en oxygène, et ainsi assurer une délivrance d'oxygène à haute concentration d'O₂ (>90%vol) à un utilisateur, typiquement un patient.

Ceci permet de limiter la consommation globale d'O₂ tout en garantissant la fourniture d'une haute concentration d'O₂ (>90%) et en réduisant significativement l'apport d'O₂ provenant de la source d'O₂.

Autrement dit, grâce à l'installation de fourniture de gaz de l'invention, on peut assurer une FiO₂ supérieure à 90% chez le patient sans avoir recours à un débit élevé d'oxygène provenant de la source d'oxygène étant donné qu'une proportion non-négligeable du flux d'oxygène fourni au patient se compose d'oxygène ayant été purifié au sein du système de purification de gaz puis recyclé dans la ligne principale d'acheminement de gaz, via la ligne de recyclage de gaz.

Réutiliser une partie de l'oxygène normalement rejeté à l'atmosphère permet de réduire la consommation globale d'oxygène et donc de limiter fortement le stress sur les réserves d'O₂ disponibles sur le site considéré, i.e. hôpital ou autre, en limitant les pertes d'oxygène.

Il est à noter que, dans le cadre de la présente invention, on utilise indifféremment la terminologie « le gaz expiré » ou « les gaz expirés » (i.e. singulier ou pluriel) pour désigner le mélange gazeux rejeté par les poumons du patient lequel contient de l'O₂, du CO₂ et généralement de la vapeur d'eau.

Selon le mode de réalisation considéré, l'installation de fourniture de gaz selon l'invention peut comprendre l'une ou plusieurs des caractéristiques suivantes :
- la source de gaz contient de l'oxygène à une concentration d'au moins 90% en volume, de préférence d'au moins 95 % en volume, avantageusement entre 98% et 100%.
- la source de gaz comprend une (ou des) bouteille de gaz sous pression ou un (ou des) récipients sous pression contenant de l'oxygène pur, i.e. concentration de 100%vol.
- alternativement, la source de gaz comprend une prise distribution d'oxygène, telle une prise murale hospitalière, alimentée en oxygène par une canalisation d'oxygène reliée fluidiquement à un réservoir de stockage d'oxygène, notamment de LOX.
- l'interface respiratoire est un masque respiratoire, en particulier un masque facial, i.e. bucco-nasal, couvrant le nez et la bouche de l'utilisateur.
- l'interface respiratoire est un masque respiratoire comprenant un orifice d'entrée d'oxygène pour fournir le gaz riche en oxygène et un orifice de sortie de gaz expiré pour évacuer le mélange gazeux CO₂/O₂ expiré par l'utilisateur.
- le masque respiratoire comprend un coussin souple venant en contact et assurant une étanchéité fluidique avec le visage de l'utilisateur.
- le masque respiratoire comprend un corps de masque, par exemple en polymère rigide, formant une coque délimitant une enceinte pour le gaz.
- le coussin souple est couplé mécaniquement au corps de masque, de préférence de manière détachable.
- le coussin souple comprend un passage central au sein duquel viennent se loger le nez et la bouche de l'utilisateur, lorsqu'il porte le masque facial.
- l'utilisateur respire le gaz contenu dans l'enceinte, c'est-à-dire inspire et expire le gaz dans l'enceinte du corps de masque.
- l'orifice d'entrée d'oxygène et l'orifice de sortie de gaz expiré sont aménagés dans le corps de masque.
- la ligne principale d'acheminement de gaz et/ou la ligne de récupération de gaz et/ou la ligne de recyclage de gaz sont ou comprennent une ou des conduites de gaz, de préférence souples, c'est-à-dire des tuyaux flexibles, par exemple en polymère.
- la ligne principale d'acheminement de gaz et/ou la ligne de récupération de gaz viennent se raccorder au corps de masque de manière à être en communication fluidique avec l'orifice d'entrée d'oxygène et l'orifice de sortie de gaz expiré.
- la ligne principale d'acheminement de gaz est raccordée fluidiquement à l'orifice d'entrée d'oxygène de l'interface respiratoire de manière à introduire du gaz respiratoire dans l'interface respiratoire.
- la ligne de récupération de gaz est raccordée fluidiquement à l'orifice de sortie de gaz expiré de l'interface respiratoire de manière à extraire du gaz respiratoire de l'interface respiratoire.
- la ligne de recyclage de gaz comprend un réservoir-tampon en communication fluidique avec la ligne principale d'acheminement de gaz.
- la ligne de recyclage de gaz est raccordée fluidiquement à la ligne principale d'acheminement de gaz par l'intermédiaire du réservoir-tampon, c'est-à-dire que la ligne de recyclage de gaz alimente le réservoir-tampon avec du gaz purifié riche en oxygène étant donné que la ligne de recyclage de gaz est raccordée fluidiquement au réservoir-tampon.
- le gaz purifié riche en oxygène acheminé par la ligne de recyclage de gaz contient une proportion (i.e. teneur) d'oxygène inférieure ou égale au gaz provenant de la source de gaz, i.e. d'oxygène pur, et acheminé par la ligne principale d'acheminement de gaz.
- le réservoir-tampon comprend un ballon flexible, un soufflet ou analogue.
- le réservoir-tampon comprend une valve d'échappement permettant d'évacuer à l'atmosphère tout excès de gaz, i.e. d'O₂, lorsque le réservoir-tampon est plein de gaz, i.e. d'oxygène.
- le réservoir-tampon est dimensionné pour contenir de 0.5 à 5 litres de gaz (à l'état non-comprimé).
- le réservoir-tampon est en outre en communication fluidique avec la ligne principale d'acheminement de gaz de manière à être alimenté en gaz respiratoire (i.e. O₂) par la ligne principale d'acheminement de gaz.
- le réservoir-tampon est configuré pour permettre d'y réaliser un mélange de gaz à partir de gaz respiratoire riche en oxygène (de préf. >98% O₂) provenant de la ligne principale d'acheminement de gaz, tel de l'oxygène pur (i.e. 100%), et de gaz purifié, i.e. gaz contenant au moins 95% d'oxygène, provenant de la ligne de recyclage de gaz
- le réservoir-tampon est configuré pour alimenter la ligne principale d'acheminement de gaz avec le gaz respiratoire, e.g. O₂ pur, ou le mélange de gaz opéré dans ledit réservoir-tampon, i.e. O₂ + gaz purifié >95% O₂.
- le système de purification de gaz comprend au moins un adsorbant présentant une sélectivité supérieure pour le CO₂ que pour l'O₂ de manière à adsorber au moins une partie du CO₂ contenu dans le mélange gazeux CO₂/O₂ expiré.
- le système de purification de gaz comprend au moins un adsorbant zéolitique.
- le système de purification de gaz comprend au moins un adsorbant choisi parmi les silicates.
- le système de purification de gaz comprend plusieurs adsorbeurs contenant chacun au moins un adsorbant, de préférence un adsorbant zéolitique ou silicate, ou leurs mélanges.
- les adsorbeurs contiennent chacun au moins un lit d'adsorbant.
- le système de purification de gaz comprend au moins un adsorbant pour éliminer le CO₂ et la vapeur d'eau (H₂O), c'est-à-dire que le CO₂ et la vapeur d'eau sont éliminés sur un même lit d'adsorbant, par exemple un lit de zéolite unique, ou sur des lits successifs d'absorbants différents, par exemple un lit de silicate et un lit de zéolite.
- les adsorbeurs sont agencés en parallèle et fonctionnent de manière alternée.
- les adsorbeurs sont ou ont une forme de cartouche.
- elle comprend trois adsorbeurs comprenant un premier adsorbeur ou cartouche, un second adsorbeur ou cartouche, et un troisième adsorbeur ou cartouche.
- un adsorbeur est en phase de d'adsorption, pendant que deux adsorbeurs sont en phase de régénération, c'est-à-dire qu'il y a toujours plus d'adsorbeurs en phase de régénération qu'en phase de d'adsorption.
- en phase d'adsorption (i.e. de production), l'adsorbant adsorbe, i.e. piège/retient, au moins une partie du CO₂ et de la vapeur d'eau (H₂O) contenue dans le mélange gazeux CO₂/O₂ expiré.
- en phase de régénération (i.e. désorption), au moins une partie du CO₂ et de la vapeur d'eau ayant été adsorbée par l'adsorbant est désorbée, i.e. libérée, et évacuée à l'atmosphère.
- la régénération par le flux d'air non-chauffé est opérée à contre-courant.
- chaque adsorbeur est configuré pour fonctionner selon des cycles d'adsorption/désorption de type PSA (Pressure Swing Adsorption), i.e. adsorption à pression modulée.
- le conduit d'air est relié fluidiquement à l'atmosphère via un orifice d'entrée d'air.
- le système de purification de gaz comprend en outre une ou plusieurs vannes de distribution de gaz agencées, sur le trajet du gaz, en amont et/ou en aval des adsorbeurs pour contrôler les entrées et les sorties de gaz desdits adsorbeurs, par exemple des vannes rotatives ou d'autres vannes.
- au moins une première vanne est agencée en amont des adsorbeurs.
- au moins une deuxième vanne est agencée en aval des adsorbeurs.
- la première vanne agencée en amont des adsorbeurs est une première vanne rotative.
- la deuxième vanne agencée en aval des adsorbeurs est une deuxième vanne rotative.
- le débit d'air ambiant non-chauffé amené par le conduit d'air sert, durant chaque phase de régénération, à régénérer l'adsorbant contenu dans deux des adsorbeurs.
- la première et/ou la deuxième vanne rotative comprennent un raccord coudé mobile en rotation dans ladite première et/ou deuxième vanne rotative.
- la première et/ou la deuxième vanne rotative comprennent un conduit d'entrée.
- la première vanne rotative comprend plusieurs ports de sortie, de préférence un premier port de sortie, un second port de sortie et un troisième port de sortie.
- la première vanne rotative comprend en outre un évent relié à l'atmosphère permettant d'évacuer du gaz pendant qu'au moins un autre adsorbeur est en phase de désorption ou régénération.
- la deuxième vanne rotative comprend plusieurs ports d'entrée, de préférence un premier port d'entrée, un second port d'entrée et un troisième port d'entrée.
- la deuxième vanne rotative comprend en outre un orifice d'entrée d'air relié fluidiquement à l'atmosphère par le conduit d'air.
- le conduit d'air est un passage, une ligne, un tuyau ou tout autre moyen d'acheminement de gaz analogue.
- la turbine, aussi appelée soufflante ou compresseur, est agencée sur le conduit d'air.
- la turbine est motorisée, c'est-à-dire équipée d'un moteur électrique.
- le premier raccord coudé est configuré pour assurer une liaison étanche avec le conduit d'entrée (i.e. à sa première extrémité) et avec les ports de sortie de la première vanne rotative (i.e. à sa seconde extrémité).
- le second raccord coudé est configuré pour assurer une liaison étanche avec le conduit de sortie (i.e. à sa deuxième extrémité) et avec les ports d'entrée de la deuxième vanne rotative (i.e. à sa première extrémité).
- les moyens de pilotage, notamment à microprocesseur(s) sont ou comprennent une unité de pilotage à microprocesseur(s).
- les moyens de pilotage comprennent un ou des microcontrôleurs.
- les moyens de pilotage comprennent un ou des microcontrôleurs agencés sur (au moins) une carte électronique.
- le ou chaque raccord coudé est couplé mécaniquement à un actionneur rotatif ou analogue piloté par les moyens de pilotage.
- l'actionneur rotatif comprend un moteur pas à pas ou analogue.
- les moyens de pilotage pilotent l'actionneur rotatif de manière à commander et/ou opérer un mouvement de rotation du ou de chaque raccord coudé.
- le système de purification de gaz comprend un premier capteur de débit agencé en amont des adsorbeurs.
- le système de purification de gaz comprend un second capteur de débit agencé en aval des adsorbeurs.
- le premier et/ou le second capteur de débit coopèrent avec les moyens de pilotage, en particulier ils leur communiquent les mesures de débit qu'ils opèrent.
- le premier et/ou le second capteur de débit est/sont un capteur de débit massique.
- les moyens de pilotage traitent les mesures de débit transmises le premier et/ou le second capteur de débit.
- les moyens de pilotage commandent l'actionneur rotatif en réponse au traitement des mesures de débit transmises le premier capteur de débit.
- le second capteur de débit est agencé sur le conduit d'air, de préférence entre la deuxième vanne rotative et la turbine.
- la turbine est commandée par les moyens de pilotage, en particulier un microcontrôleur.
- la turbine est commandée par les moyens de pilotage de manière à générer un débit circulant dans le conduit d'air.
- l'installation comprend en outre des moyens alimentation électrique alimentant les composants de l'installation ayant besoin de courant électrique pour fonctionner, notamment les moyens de pilotage, la ou les vannes de distribution et/ou la turbine motorisée.
- le système de purification de gaz est agencé dans un boitier rigide.
- la turbine est agencée entre l'orifice d'entrée d'air et le second capteur de débit massique. L'association de la turbine et du second capteur de débit massique qui sont alimentés et contrôlés par la carte de commande et pilotés par le microcontrôleur, forme un contrôleur de débit massique.
- les moyens de pilotage, en particulier le microcontrôleur, sont configurés pour déterminer une consigne de débit Q, c'est-à-dire fixer un débit cible Q devant circuler dans le conduit d'air.
- les moyens de pilotage, en particulier le microcontrôleur, sont configurés pour contrôler la turbine pour délivrer un flux d'air mesure dont le débit est égale ou approximativement égal à la consigne de débit Q.
- le second capteur de débit est configuré pour mesurer le débit généré par ladite turbine et contrôler la turbine en réponse à la mesure de débit opérée.
- la source de gaz contient de l'oxygène gazeux.
- la source de gaz est une bouteille de gaz ou une prise d'O₂ murale alimentée en oxygène par une canalisation de gaz, telle réseau de gaz d'un bâtiment hospitalier.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de fourniture d'O₂ selon la présente invention.
Fig. 2 schématise un mode de réalisation de l'architecture d'une unité de purification des gaz expirés de l'installation de Fig. 1.
Fig. 3 représente une vanne de rotation servant à distribuer les gaz dans l'unité de purification des gaz expirés de Fig. 2.
Fig. 4 et Fig. 5 représentent d'autres configurations de l'unité de purification des gaz expirés de Fig. 2 suite à une action des vannes rotatives selon Fig. 3.

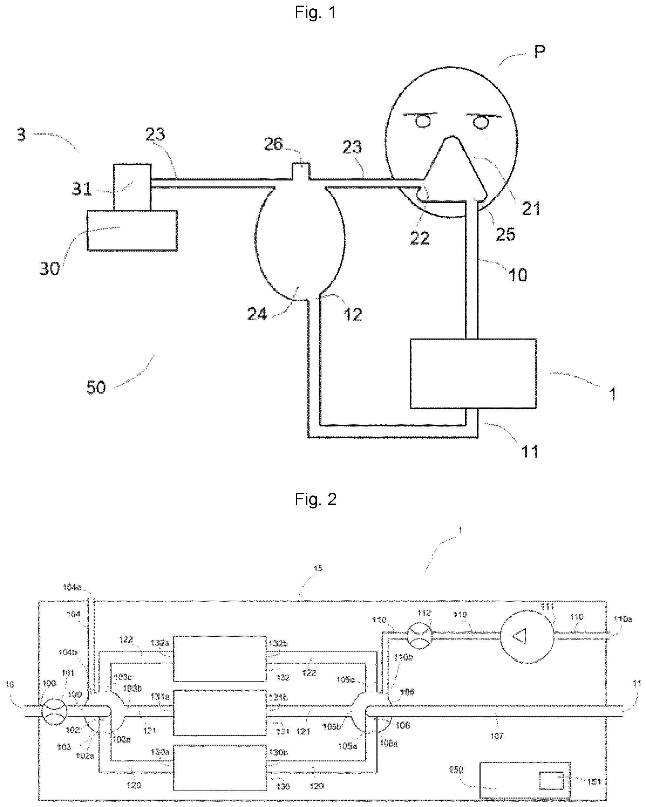

Fig. 1 schématise un mode de réalisation d'une installation 50 de fourniture d'un mélange gazeux riche en O₂ selon la présente invention comprenant une source de gaz 3 pour fournir un gaz respiratoire contenant de l'oxygène, de préférence de l'oxygène pur, une ligne principale d'acheminement de gaz 23, aussi appelée circuit patient, une interface respiratoire 21, tel un masque facial, pour administrer le gaz respiratoire à l'utilisateur, lors de ses phases inspiratoires, et un système ou unité de purification 1 des gaz expirés.

La source de gaz 3 peut être par exemple une prise d'O₂ murale 30, comme illustré en Fig. 1, ou une bouteille d'oxygène (non montrée).

Dans le mode de réalisation illustré en Fig. 1, la source de gaz 3 est une prise d'O₂ murale 30 située dans le lieu de soin, tel un bâtiment hospitalier, et connectée à une réserve d'O₂, par exemple un réservoir de stockage d'O₂ liquide (LOX) de plusieurs milliers de litres, située à l'extérieure du site et alimentant le réseau de canalisations d'oxygène agencé dans le bâtiment hospitalier ou analogue. Il peut être prévu un vaporiseur de gaz, en sortie du réservoir de stockage de LOX, permettant de vaporiser le LOX et obtenir de l'oxygène gazeux (GOX) qui alimente le réseau de canalisations d'oxygène.

La prise murale 30 est équipée d'un débitmètre à bille 31 permettant un réglage par l'utilisateur d'un débit de gaz donné, typiquement entre 1 et 15 L/min. De préférence, une alimentation constante en gaz est délivrée par la source de gaz 3.

Dans le cadre de la présente invention, le débit d'O₂ réglé est préférentiellement un débit faible, à savoir par exemple de l'ordre de 1 L/min, de manière à limiter la consommation d'oxygène provenant de la source de gaz 3, e.g. la prise murale 30.

La ligne principale d'acheminement de gaz 23, i.e. le circuit patient, comprend un tuyau flexible qui relie fluidiquement la source de gaz 3, via le débitmètre à bille 31, à l'orifice ou port d'entrée 22 de l'interface respiratoire 21 de manière à alimenter le patient P en O₂, par exemple un masque facial.

La ligne principale d'acheminement de gaz 23 comprend par ailleurs un réservoir-tampon 24 en communication fluidique avec le lumen de ladite ligne principale d'acheminement de gaz 23, et servant de réserve de gaz respiratoire, i.e. d'oxygène, au patient P.

Typiquement, le réservoir-tampon 24 est alimenté en oxygène provenant par la ligne principale d'acheminement de gaz 23 et en gaz purifié recyclé provenant de la ligne de recyclage 11, de préférence de l'oxygène ayant une pureté de 95% ou plus, comme expliqué ci-après.

Lorsque le patient P respire, le réservoir-tampon 24 qui est rempli de gaz, satisfait la demande instantanée du patient P en lui fournissant au moins une partie du gaz qu'il contient, à savoir de l'oxygène pur ou quasi-pur.

Par ailleurs, le gaz expiré par le patient P ressort de l'interface respiratoire 21 par un port ou orifice de sortie 25 aménagé dans l'interface respiratoire 21, avant d'être récupéré et convoyé par une ligne de récupération de gaz 10 formant un circuit expiratoire, tel un conduit ou tuyau de gaz flexible, connecté fluidiquement au port de sortie 25 de l'interface respiratoire 21.

Cette ligne de récupération de gaz 10 permet de récupérer et acheminer au moins une partie du mélange gazeux CO₂/O₂ expiré par l'utilisateur se retrouvant dans l'interface respiratoire 21, lors des phases expiratoires de l'utilisateur P. Ce gaz expiré peut aussi contenir un ou d'autres composés gazeux, en particulier de la vapeur d'eau (H₂O).

Dans tous les cas, le mélange gazeux CO₂/O₂ expiré contient encore une forte proportion d'O₂, par exemple au moins 90 à 95% d'O₂ alors que sa teneur en dioxyde de carbone (et en vapeur d'eau) issu de la respiration cellulaire et des échanges pulmonaires est relativement faible, par exemple de l'ordre de 5 % vol.

Ce flux gazeux de mélange CO₂/O₂ est acheminé par la ligne de récupération de gaz 10 jusqu'à une unité de purification 1 des gaz expirés au sein de laquelle le CO₂ et éventuellement la vapeur d'eau sont épurés/éliminés, notamment adsorbés, i.e. captés, par un (ou des) lit de d'adsorbants.

Le gaz purifié produit par cette unité de purification 1 contient essentiellement de l'oxygène et des quantités faibles, voire négligeables, de CO₂ et éventuellement de vapeur d'eau (i.e. humidité). Dans tous les cas, le gaz purifié a, après purification, une teneur en oxygène supérieure à celle du gaz expiré.

Autrement dit, le système de purification de gaz 1 est alimenté en mélange gazeux CO₂/O₂ expiré (i.e teneur O₂ < 95% env.) par la ligne de récupération de gaz 10 et permet d'éliminer la majeure partie du CO₂ et de la vapeur d'eau contenue dans le mélange gazeux CO₂/O₂ expiré et obtenir un gaz purifié contenant essentiellement de l'oxygène (e.g. teneur O₂ > 99% env.).

En aval du système de purification de gaz 1, une ligne de recyclage de gaz 11 reliée fluidiquement au système de purification de gaz 1 et par ailleurs à la ligne principale d'acheminement de gaz 23, par exemple via le réservoir-tampon 24, permet de recueillir et acheminer le gaz purifié contenant essentiellement de l'oxygène (e.g. teneur O₂ > 99% env.) provenant du système de purification de gaz 1 et le réinjecter dans la ligne principale d'acheminement de gaz 23, c'est-à-dire dans le flux d'oxygène provenant de la source d'oxygène 3, telle une prise murale 30.

La quantité de composés CO₂ et vapeur d'eau résiduels dans le gaz purifié provenant du système de purification de gaz 1 sont négligeables, typiquement <0.1% vol., c'est-à-dire que le gaz purifié est essentiellement constitué d'O₂ quasi-pur.

Le gaz purifié, i.e. oxygène, est acheminé par la ligne de recyclage de gaz 11 jusqu'au réservoir 24 et y pénètre via un port d'entrée 12 auquel est raccordée fluidiquement la ligne de recyclage de gaz 11.

Selon l'invention, le système de purification de gaz 1 constitue donc une unité d'épuration de gaz expirés permettant de purifier et recycler ensuite les gaz expirés riche en O₂ en les débarrassant du CO₂ et éventuellement de la vapeur d'eau (H₂O) qu'ils contiennent, au lieu de les rejeter à l'atmosphère, comme c'est habituellement le cas, et ainsi gaspiller l'oxygène qui s'y trouve encore en grande quantité, typiquement au moins 90% vol. environ.

Récupérer la majeure partie de l'O₂ formant le composé fortement majoritaire dans les gaz expirés par le patient P, et la recycler après purification, permet d'éviter son gaspillage, et donc ainsi de limiter le débit d'oxygène fourni par la source d'O₂ 3.

Par exemple, dans les cas les plus extrêmes, la consommation métabolique d'O₂ d'un être humain est de l'ordre de 0.5 L/min. Autrement dit, si un patient a une ventilation minute de 10 L/min, i.e. inhale 10L de gaz en 1 minute, d'O₂ pur, c'est-à-dire à teneur de 100% d'O₂, alors 9.5 L d'O₂ seront expirés, le reste (0.5 L) sera constitué de CO₂ et de vapeur d'eau. Dès lors, le système de recyclage 1 de l'invention permet de récupérer environ 9.5L d'O₂. En réglant un débit de 1 L/min sur le débitmètre à bille 31 de la source d'O₂ 3 et en recyclant l'O₂ contenu dans le gaz expiré en le mélangeant avec l'O₂ provenant de la source d'O₂ 3, le débit d'O₂ obtenu excède alors la ventilation minute du patient.

Préférentiellement, le réservoir-tampon 24 est muni d'une valve d'échappement 26 permettant d'évacuer à l'atmosphère ambiante tout excès d'O₂ lorsque le réservoir-tampon 24 est plein d'oxygène.

Fig. 2 schématise un mode de réalisation du système de purification de gaz 1, aussi appelé « unité d'épuration de gaz expiré » par le patient, faisant partie de l'installation 50 de fourniture de mélange gazeux respiratoire, typiquement de l'oxygène, selon la présente invention, illustrée sur la Fig. 1.

Comme déjà expliqué et davantage détaillé ci-après, le système de purification de gaz 1 selon la présente invention sert à purifier le gaz expiré par le patient lequel contient de l'O₂, du CO₂ et généralement de la vapeur d'eau, par élimination du CO₂ et de la vapeur d'eau présente, de manière à obtenir un gaz purifié contenant une forte proportion d'oxygène, typiquement au moins 90 à 95% vol. d'oxygène.

Le système de purification de gaz 1 ou unité d'épuration de gaz expiré comprend des moyens de pilotage 150, 151, telle une carte 150 de commande électronique, i.e. une carte électronique, et une unité de contrôle 151 à microprocesseur(s), typiquement un microcontrôleur.

Tous les éléments électromécaniques du système de purification 1 sont alimentés électriquement et commandés par les moyens de pilotage 150, 151. Les moyens de pilotage 150, 151 sont eux-mêmes alimentés électriquement par une source de courant électrique (non montrée), par exemple une liaison au courant du secteur (1 10/220V) de type cordon électrique et prise de raccordement, ou une (ou des) batterie d'alimentation électrique, de préférence rechargeable, et/ou un transformateur de courant.

La carte de commande 150 intègre préférentiellement l'unité de contrôle 151 à microprocesseur et est configurée pour commander et par ailleurs analyser les signaux, notamment les mesures, provenant des différents composants de l'unité d'épuration de l'air expiré 1, tels que vannes, pompe, capteurs...

Les moyens de pilotage 150, 151, notamment la carte de commande 150, et les autres composants de l'unité de purification 1 sont agencés, i.e. intégrés, dans une carcasse ou boitier 15 externe rigide, par exemple en polymère.

L'unité d'épuration de gaz expiré 1 comprend un conduit ou passage d'entrée 100 avec orifice d'entrée qui est relié fluidiquement à la ligne de récupération de gaz 10, aussi appelée circuit expiratoire, c'est-à-dire un conduit de gaz ou analogue, servant à recueillir le gaz expiré par le patient (i.e. mélange O₂, CO₂, vapeur d'eau...) qui est convoyé par la ligne de récupération de gaz 10.

La ligne de récupération de gaz 10 vient se raccorder fluidiquement, par son extrémité aval, au conduit d'entrée 100 via un système de raccordement comprenant par exemple des connecteurs réciproques de type mâle/femelle, permettant d'assurer une connexion mécanique et fluidique, et par ailleurs à l'interface respiratoire 21, c'est-à-dire un masque facial par exemple, par son extrémité amont, afin d'y prélever le gaz expiré par le patient P.

Autrement dit, l'entrée du gaz expiré par le patient contenant majoritairement de l'O₂, (par exemple 90-95% vol. env.), ainsi que du CO₂ et généralement de la vapeur d'eau, se fait dans la portion amont ou portion d'entrée du conduit d'entrée 100 de l'unité d'épuration de gaz expiré 1, dans lequel est agencé par ailleurs un premier capteur de débit 101, typiquement un capteur de débit massique.

En aval du capteur de débit 101, le conduit d'entrée 100 est fluidiquement connecté à un raccord coudé 102 mobile, c'est-à-dire se mouvant de façon circulaire dans une première vanne rotative 103 munie de trois ports de sortie 103a-103c, à savoir un premier port de sortie 103a, un second port de sortie 103b et un troisième port de sortie 103c, ainsi que d'un évent 104b relié à l'atmosphère. Le raccord coudé 102 est configuré pour assurer une liaison étanche avec le conduit d'entrée 100 à sa première extrémité 102b ainsi qu'avec l'un ou l'autre des ports de sortie 103a-103c de la vanne rotative 103, via sa seconde extrémité 102a, par exemple le premier port de sortie 103a de la vanne rotative 103.

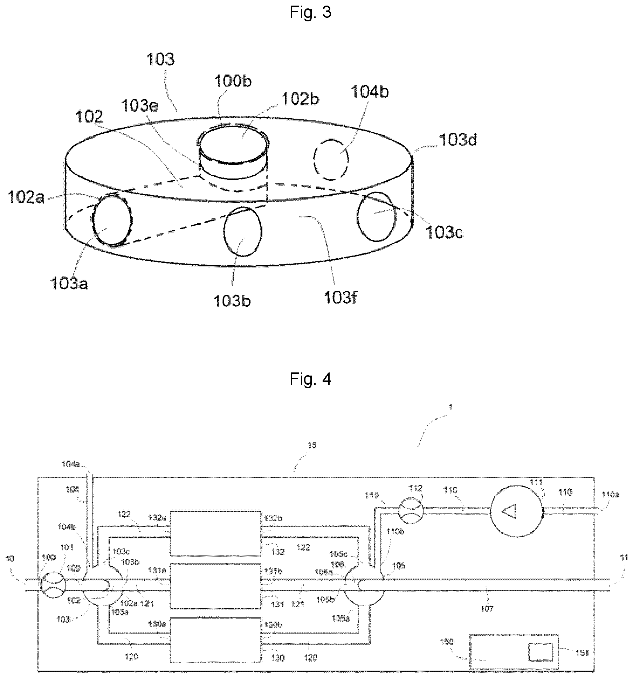

Fig. 3 schématise un mode de réalisation de la vanne rotative 103 montrant le raccord coudé 102 avec ses deux extrémité 102b, 102a.

L'ensemble est configuré pour qu'une étanchéité entre le conduit d'entrée 100, en particulier son extrémité 100b (représentée en pointillé) et la première extrémité 102b du raccord coudé 102 soit possible tout en laissant au raccord coudé la possibilité d'opérer des mouvements rotatifs dans l'enceinte 103d de la vanne rotative 103. Pour ce faire, on peut opérer un ajustement précis et utiliser des joints toriques (non représentés). On configure de la même manière, la seconde extrémité 102a, le premier port de sortie 103a et l'évidement 103e de l'enveloppe 103d de la vanne rotative 103 et la section résultant du raccord coudé 102.

Dans le mode de réalisation de Fig. 3, il existe une relation fluidique entre l'extrémité 100b du conduit d'entrée 100, et le port de sortie 103a de la vanne rotative 103.

Dans le même temps, l'évidement 103f interne à la vanne rotative 103, en particulier de son enveloppe 103d, met en relation fluidique les second et/ou troisième ports de sortie 103b, 103c et l'évent 104b.

Enfin, le raccord coudé 102 peut être mécaniquement couplé à un actionneur rotatif (non représenté), tel qu'un moteur pas à pas, pouvant être piloté par le microcontrôleur 151 afin d'engager un mouvement de rotation du raccord coudé 102 et changer la configuration fluidique de la vanne rotative 103. Par exemple, un mouvement de rotation peut mettre en relation l'extrémité 100b du conduit d'entrée 100 et le second port de sortie 103b de la vanne rotative 103. Les premier et troisième port 103a, 103c sont alors en relation fluidique avec l'évent 104b.

De façon analogue, le microcontrôleur 151 peut commander une mise en relation fluidique entre l'extrémité 100b du conduit d'entrée 100 et le troisième port de sortie 103c de la vanne rotative 103. Les premier et second ports de sortie 103a, 103b de la vanne rotative sont alors en relation fluidique avec l'évent 104b.

Sur la Fig. 2, il apparaît que le premier port de sortie 103a de la vanne rotative est fluidiquement connecté à un premier conduit 120, tandis que les second et troisième ports de sortie 103b, 103c de la vanne rotative 103 sont respectivement connectés à un second conduit 121 et troisième conduit 122. Par ailleurs, l'évent 104b est fluidiquement connecté à un conduit d'échappement 104 traversant la carcasse 15 de l'unité d'épuration de gaz expiré 1 pour échapper à l'atmosphère ambiante, via le port de sortie 104a. Le conduit d'entrée 100 est donc fluidiquement connecté au premier conduit 120, alors que les second et troisième conduits 121, 122 sont fluidiquement connectés à conduit d'échappement 104 et par prolongement à l'atmosphère ambiante via le port de sortie 104a.

Agencée dans le premier conduit 120 se trouve une première cartouche 130, c'est-à-dire un premier adsorbeur, présentant un premier port amont 130a (e.g. un orifice d'entrée) et un premier port aval 130b (e.g. un orifice de sortie). De façon similaire, une seconde cartouche ou deuxième adsorbeur 131 se trouve agencé dans le second conduit 121 et présente un second port amont 131a et un second port aval 131b, et une troisième cartouche ou troisième adsorbeur 132 se trouve agencé dans le troisième conduit 122 et présente un troisième port amont 132a et troisième port aval 131b.

Les première, seconde et troisième cartouches 130, 131, 132 sont des récipients, par exemple en métal, et sont agencées en parallèle les unes des autres sur le trajet du gaz véhiculé par lesdits premier, second et troisième conduits 120-122, respectivement.

Les première, seconde et troisième cartouches 130-132 renferment chacune un ou plusieurs adsorbants ou tamis moléculaire(s), par exemple agencés en lit(s) d'absorbant(s), notamment un (ou des) adsorbant de type zéolite, telle une zéolite 13X, et/ou de type silicate. Par exemple, chaque cartouche 130-132 peut contenir environ 500g de zéolite 13X et de silicates.

L'adsorbant est généralement formé de particules, notamment de billes ou analogues. Dans tous les cas, on choisit un (des) adsorbant(s) qui permet d'adsorber ou piéger préférentiellement le CO₂, voire aussi la vapeur d'eau, c'est-à-dire présentant une sélectivité plus élevée pour le CO₂ (et l'eau) que pour l'oxygène, de manière à produire un flux purifié contenant une concentration en oxygène supérieure à celle dans le gaz expiré par le patient, c'est-à-dire avant sa purification. L'adsorbant sert donc à concentrer le flux en oxygène en éliminant au moins une partie des autres espèces qui s'y trouvent, à savoir le dioxyde de carbone et l'eau.

Bien entendu, d'autres matériaux adsorbants, tels que des MOF (Metal Organic Framework) ou autres peuvent être être utilisés en remplacement ou supplément pour augmenter la capacité et/ou sélectivité d'adsorption du CO₂.

Lorsque le gaz a traversé l'une ou l'autre des première, seconde et troisième cartouches 130-132 (i.e. adsorbeurs), il ressort sous formé de gaz purifié par l'un des orifices de sortie ou ports avals 130b-132b, avant d'être acheminé par l'un ou l'autre des premier, second et troisième conduits 120-122 jusqu'à une seconde vanne rotative 105.

Plus précisément, les portions avals des premier, second et troisième conduits 120-122 sont respectivement connectées à un premier port d'entrée 105a, second port d'entrée 105b et troisième port d'entrée 105c de ladite seconde vanne rotative 105.

Cette seconde vanne rotative 105 est dans son fonctionnement en tout point similaire à la première vanne rotative 103, c'est-à-dire qu'elle présente un raccord coudé 106 permettant de mettre en relation, en fonction de son positionnement par l'unité de contrôle 151, l'un des ports d'entrée de la second vanne rotative 105, par exemple le premier port d'entrée 105a, avec un conduit de sortie 107 prolongé par le conduit d'échappement 11 connecté à l'unité d'épuration de gaz expiré 1, alors que les second et troisième ports d'entrée 105b et 105c sont en relation fluidique avec un port d'admission 110b de ladite seconde vanne rotative 105.

Ce port d'admission 110b constitue la principale différence entre les première vanne rotative 103 et la seconde vanne rotative 105 car celui-ci est connecté à un conduit d'air 110 relié fluidiquement à l'atmosphère par un orifice d'entrée d'air 110a, dans lequel sont agencés une turbine 111, aussi appelée soufflante ou compresseur, et un autre capteur de débit massique, appelé second capteur de débit 112.

La turbine 111 est agencée entre l'orifice d'entrée d'air 110a et le second capteur de débit massique 112. L'association de la turbine 111 et du second capteur de débit massique 112, qui sont alimentés et contrôlés par la carte de commande 150 et pilotés par le microcontrôleur 151, forme un contrôleur de débit massique.

Plus précisément, le microcontrôleur 151 peut déterminer une consigne de débit Q, c'est-à-dire fixer un débit cible Q devant circuler dans le conduit d'air. Le microcontrôleur 151 contrôle alors la turbine 111 de telle manière à ce que le second capteur de débit 112, qui mesure le débit généré par ladite turbine 111, mesure un débit proche ou égal au débit cible Q fixé par le microcontrôleur 151.

Le gaz expiré par le patient P, qui contient une haute teneur en O₂ (e.g. >90% vol) mais aussi du CO₂ et généralement de la vapeur d'eau, emprunte, la ligne de récupération de gaz 10 formant un circuit expiratoire, avant d'être admis dans l'unité d'épuration de gaz expiré 1 de la Fig. 2, via le conduit d'entrée 100.

Du fait des configurations pneumatiques imposées par les première et seconde vannes rotatives 103, 105, le conduit d'entrée 100 est fluidiquement relié au conduit de sortie 107 via le premier conduit 120, dans lequel est agencé la première cartouche 130 ou adsorbeur.

Ainsi, le gaz empruntant le conduit d'entrée 100 est dirigé dans la première cartouche 130, via son premier port aval 130a, pour y être épuré du CO₂ et de la vapeur d'eau, c'est-à-dire que le ou les adsorbants remplissant la première cartouche ou adsorbeur 120 captent, c'est-à-dire retiennent/piègent les molécules de CO₂ et de vapeur d'eau, en laissant passer l'O₂, c'est-à-dire sans l'adsorber.

Par exemple, la capacité et/ou la sélectivité d'adsorption du CO₂ et de la vapeur d'eau des zéolites, en particulier la zéolite 13X, ou bien des silicates est supérieure à la capacité/sélectivité d'adsorption de l'O₂ de sorte que ces composés CO₂ et vapeur d'eau soient préférentiellement piégés/adsorbés, alors que l'oxygène peut traverser librement le lit d'adsorbant sans y être retenu ou très peu.

Ainsi, le gaz purifié sortant de la première cartouche 130, via le premier port aval, 130b est épuré du CO₂ et de la vapeur d'eau, et contient de préférence plus de 95% d'oxygène. Après sa sortie de l'unité d'épuration des gaz expirés 1, il est récupéré et acheminé par la ligne de recyclage de gaz 11 formant un circuit d'échappement, en ayant au préalable transité dans le premier conduit 120 situé en aval de la première cartouche 130 et le conduit de sortie 107.

Ce gaz purifié peut être alors réinjecté dans la ligne principale d'acheminement de gaz 23, via le réservoir 24 dans lequel il pénètre via le port additionnel 12.

Ce flux de gaz purifié qui contient de l'oxygène à haute concentration (e.g. >95%) se mélange donc, au sein du réservoir 24, avec de l'oxygène (e.g. 100%) provenant de la source d'oxygène 3 de manière à obtenir un mélange d'oxygène de très haute pureté (i.e. proche de 100% vol), qui peut être ensuite acheminé jusqu'au patient P et lui être administré via l'interface respiratoire 21 afin d'être ré-inhalé.

Autrement dit, le flux de gaz purifié vient se substituer à une partie de l'oxygène provenant de la source d'oxygène 3, ce qui permet de limiter la consommation d'oxygène provenant de la source d'oxygène 3 et par ailleurs le gaspillage d'oxygène en recyclant le gaz expiré qui serait normalement rejeté à l'atmosphère.

L'installation de fourniture 50 de gaz respiratoire, i.e. d'oxygène, selon l'invention comprend un système de purification de gaz 1 qui fonctionne de manière cyclique, du fait de l'utilisation d'adsorbant(s) qui nécessite d'être régénéré afin de désorber/libérer les composés ayant été piégés, à savoir le CO₂ et la vapeur d'eau.

Plus précisément, le patient respire le gaz riche en oxygène en alternant phases inspiratoires avec inhalation d'oxygène et phases expiratoires avec rejet de mélange CO₂/O₂/H₂O.

Dans l'unité d'épuration des gaz expirés, les composés CO₂ et la vapeur d'eau sont adsorbés, c'est-à-dire retenus par l'adsorbant. Or, celui-ci possède une capacité maximale d'adsorption au-delà de laquelle l'adsorbant (ou les adsorbants) est saturé, c'est-à-dire ne peut plus capter les molécules de CO₂, voire celles d'eau, qui peuvent alors traverser l'adsorbant avec l'oxygène et se retrouver dans le gaz « purifié ».

Afin d'éviter une telle situation de saturation de l'adsorbant, et donc que le patient P ne puisse ré-inhaler du CO₂ et donc éviter une situation dangereuse pour le patient, il convient de régénérer périodiquement chaque adsorbant afin de le débarrasser des composés qui y sont adsorbés/retenus, en particulier le CO₂.

Selon un mode de réalisation, la capacité maximale d'adsorption de la première cartouche 130, c'est-à-dire le volume maximal de CO₂ pouvant y être retenu, i.e. capté, est connue et mémorisée au sein du microcontrôleur 151 ou ailleurs.

En mesurant le volume total de gaz expiré ayant été admis dans l'unité d'épuration des gaz expirés 1, au moyen du premier capteur de débit 101, et en considérant une concentration de CO₂ de l'ordre de 5% vol., le microcontrôleur 151 est en mesure de déterminer que la première cartouche 130 est proche d'arriver à saturation. On procède de la même manière pour les autres cartouches 131, 132.

Bien entendu, selon d'autres modes de réalisation, l'évaluation du taux de saturation des cartouches 130-132 pourrait être opérée autrement, par exemple en mesurant la température des adsorbants ou encore en intégrant une mesure de teneur en CO₂ dans le conduit de sortie 107. Selon encore un autre mode de réalisation, la régénération des cartouches 130-132 pourrait aussi être opérée après une durée donnée ou de toute autre façon.

En considérant que la première cartouche 130 parvient à saturation, le microcontrôleur 151 commande la première vanne rotative 103 et la seconde vanne rotative 105 afin de basculer sur une cartouche non saturée et/ou ayant été régénérée, permettant de continuer à épurer les gaz expirés par le patient P.

Ceci est illustré en Fig. 4 où la première vanne rotative 103 réalise une connexion fluidique entre le conduit d'entrée 100 et le second conduit 121, alors que dans le même temps, la seconde vanne rotative 105 réalise une connexion fluidique entre le même second conduit 121, en particulier sa portion aval située en aval du second port aval 131b de la seconde cartouche 131, et le conduit de sortie 107. Ainsi, les gaz empruntant le circuit d'échappement 11 peuvent être introduit dans le réservoir 24 via le port d'entrée 12. Dans un telle configuration, la première cartouche 130 est saturée et doit être régénérée.

Classiquement, lors d'une régénération d'un lit d'adsorbant, un gaz sec c'est-à-dire dépourvu d'humidité, i.e. de vapeur d'eau, par exemple du N₂ issu d'une bouteille, est chauffé à haute température, par exemple 300°C et traverse l'adsorbant. En gérant de façon appropriée le profil de température, il est possible de régénérer l'adsorbant rapidement, par exemple de régénérer l'adsorbant en un temps égal au temps ayant mené à la saturation de l'adsorbant dans la cartouche considérée.

Toutefois, dans le cadre de la présente invention, une régénération de ce type n'est pas adaptée du fait de l'absence, en général, d'une source de gaz sec sur site d'utilisation, typiquement en hôpital ou analogue. De plus, le fait de devoir porter le gaz à haute température entraîne une complexité technique et donc induit un surcoût ainsi qu'une consommation électrique importante.

Dès lors, dans le cadre de la présente invention, on préfère assurer une régénération par le biais d'air ambiant non réchauffé. En procédant ainsi, la régénération ne peut être que partielle, c'est-à-dire que seule une partie des composés CO₂ et vapeur d'eau captés par l'adsorbant, est désorbée/libérée.

La régénération par le flux d'air non-chauffé est opérée à contre-courant, c'est-à-dire dans le sens opposé au sens de circulation du gaz pendant l'étape d'adsorption, c'est-à-dire de production d'oxygène.

Ainsi, si la capacité d'adsorption en CO₂ d'une zéolite de type 13X fraîche est de 60 ml/g, il apparaît que la régénération par de l'air ambiant non réchauffé conduira à une désorption de l'ordre de 70% d'une désorption totale, c'est-à-dire à une capacité de l'ordre de 40 ml/g. Toutefois, cette diminution volontaire de la capacité d'adsorption est prise en compte par le microcontrôleur 151 afin de déterminer, en mesurant le volume total de gaz expiré ayant été admis dans l'unité d'épuration des gaz expirés 1 via le premier capteur de débit 101, que la première cartouche 130 parvient à saturation.

De plus, le temps de désorption pour aboutir à un équilibre, c'est-à-dire ne plus être en mesure de désorber le CO₂ et la vapeur d'eau résiduels, est rallongé par exemple un temps doublé.

Afin de prendre en compte cette durée de désorption rallongée, l'unité d'épuration des gaz expirés 1 présente trois cartouches ou adsorbeurs 130-132, à savoir la première 130, la seconde 131 et la troisième cartouche 132, qui sont agencées en parallèle et fonctionnent de manière alternée. Ainsi, sur Fig. 4, la première cartouche 130 est saturée, la seconde cartouche 131 recueille les gaz expirés par le patient P pour purifier lesdits gaz expirés du CO₂, et la troisième cartouche 132 n'a pas encore été exposée au gaz expirés.

Lorsque le microcontrôleur 151 a déterminé que la première cartouche 130 arrivait à saturation et contrôlé les première et seconde vannes rotatives 103, 105 pour acheminer les gaz expirés par le patient P dans la seconde cartouche 131, le microcontrôleur pilote la turbine 111 de sorte qu'un débit d'air emprunte le conduit d'air 110, i.e. de l'orifice d'entrée d'air 110a au port d'admission 110b, et que ce débit soit d'environ 10L/min, mesuré par le second capteur de débit 112.

Comme illustré en Fig.4, le débit d'air circulant dans le conduit d'air 110 débouche dans la seconde vanne rotative 105 via le port d'admission 110b. Du fait de la configuration de la seconde vanne rotative 105, le débit d'air va emprunter le premier conduit 120 et le troisième conduit 122 en sortant de la seconde vanne rotative 105 via respectivement ses premier et troisième ports d'entrée 105a, 105c. Ainsi, le débit se propageant dans le premier conduit 120 va pénétrer la première cartouche 130 via le premier port aval 130b et charrier une partie du CO₂ et de la vapeur d'eau que ladite cartouche 130 contient pour en sortir via le premier port amont 130a.

En d'autres termes, le débit d'air sortant de la première cartouche 130 via le premier port amont 130a est enrichi en CO₂ et en vapeur d'eau.

Sur Fig. 4, le débit se propageant dans la portion amont du premier conduit 120 débouche dans la première vanne rotative 103 via son premier port de sortie 103a et emprunte alors le conduit d'échappement 104, c'est-à-dire en sortant de la première vanne rotative 103 via l'évent 104b puis en est évacué à l'air ambiant via le port de sortie 104a du conduit d'échappement 104. De même, une portion du débit d'air va emprunter le troisième conduit 122 pour traverser la troisième cartouche 132 puis être acheminé dans le conduit d'échappement 104 à travers la communication fluidique opéré par la première vanne rotative 103, en particulier le troisième port de sortie 103c et l'évent 104b. Dans ce cas, le gaz sortant de la troisième cartouche 132 en son troisième port amont 132a n'est pas enrichi en CO₂.

Du fait d'un dimensionnement identique des premier et troisième conduits 120, 122 et des première et troisième cartouches 130, 132, les débit d'air circulant dans les premier et troisième conduits 120, 122 sont égaux, par exemple de 5 L/min.

Alors que les première et troisième cartouche 130, 132 subissent un cycle de régénération (sans effet sur la troisième cartouche 132 qui n'a pas encore été exposée au gaz expirés par le patient P), le microcontrôleur 151 peut déterminer que la seconde cartouche 131 parvient à saturation. Ainsi, le microcontrôleur 151 commande la première vanne rotative 103 et la seconde vanne rotative 105 afin de basculer sur une cartouche non saturée, permettant de continuer à épurer les gaz expirés par le patient P, à savoir la troisième cartouche 132.

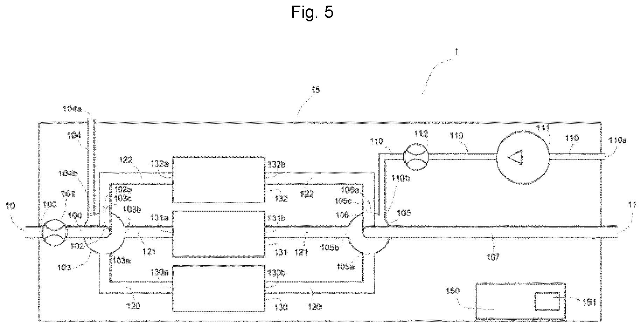

Ceci est illustré en Fig. 5 où la première vanne rotative 103 réalise une connexion fluidique entre le conduit d'entrée 100 et le troisième conduit 122 alors que, dans le même temps, la seconde vanne rotative 105 réalise une connexion fluidique entre le même troisième conduit 122, en particulier sa portion aval située en aval du troisième port aval 132b de la troisième cartouche 132, et le conduit de sortie 107. Ainsi, les gaz empruntant le circuit d'échappement 11 peuvent être introduit dans le réservoir 24 via le port d'entrée 12.

Dans un telle configuration, le débit d'air circulant dans le conduit d'air 110 continue d'emprunter le premier conduit 120 car d'une part, une connexion fluidique existe toujours entre le port d'admission 110b du premier port d'entrée 105a de la seconde vanne rotative 105, connecté au premier conduit 120, et d'autre part le premier port de sortie 103a de la première vanne rotative 103 et l'évent 104b, connecté au conduit d'échappement 104.

Comme visible en Fig. 5, une portion du débit d'air circulant dans le conduit d'air 10 emprunte le second conduit 121 du fait de la nouvelle communication fluidique opérée par la seconde vanne rotative 105 et la première vanne rotative 103.

Plus précisément, une connexion fluidique existant entre le port d'admission 110b du second port d'entrée 105b de la seconde vanne rotative 105, connecté au second conduit 121, et d'autre part le second port de sortie 103b de la première vanne rotative 103 et l'évent 104b, connecté au conduit d'échappement 104.

Ainsi, comme précédemment, la seconde cartouche 131 entame une phase de régénération, c'est-à-dire que le débit gazeux empruntant la portion amont du second conduit 121, situé en amont du second port amont 131a de la seconde cartouche 131, est lui-même enrichi en CO₂ et vapeur d'eau. Lorsque le microcontrôleur 151 détermine que la troisième cartouche 132 arrive elle-même à saturation le microcontrôleur 151 commande la première vanne rotative 103 et la seconde vanne rotative 105 afin de basculer sur une cartouche non saturée, permettant alors de continuer à épurer les gaz expirés par le patient P, à savoir la première cartouche 130.

En supposant que chaque cartouche devient saturée en 30 min environ pour ensuite être régénérée en 60 min environ, il apparaît que la première cartouche 130 a été exposée au débit d'air issu du conduit d'air 110 pendant deux phases successives de 30 min, illustrées sur les Fig. 4 et Fig. 5, et est donc maintenant régénérée, alors que la seconde cartouche 131 n'aura été exposée au même débit d'air que pendant 30 min et donc partiellement régénérée, à savoir à moitié régénérée.

En basculant sur la configuration de la Fig. 2 et en dirigeant les gaz expirés par le patient P dans la première cartouche 130, maintenant régénérée, le cycle de capture de CO₂ et de vapeur d'eau peut être poursuivi.

Du fait des configurations des première et seconde vannes rotatives 103, 105, la seconde cartouche 131 poursuit son cycle de régénération, tandis que la troisième cartouche 132 commence ce même cycle de régénération. Une fois la première cartouche 130 arrivant à saturation, le microcontrôleur 151 configure l'unité d'épuration des gaz expirés 1 comme illustré en Fig. 4, afin de diriger les gaz expirés par le patient P dans la seconde cartouche 131, nouvellement régénérée tandis que la première cartouche 130 commence sa première phase de régénération et la troisième cartouche 132 entame sa seconde phase de régénération.

Il apparaît qu'en basculant de façon cyclique entre les configurations illustrées en Fig. 2, Fig. 4 et Fig. 5, l'unité d'épuration des gaz expirés 1 est en mesure de réaliser de façon continue l'épuration des gaz expirés par le patient P tout en assurant une régénération des cartouches d'adsorbant.

Autrement dit, dans le cadre de l'invention, lorsque 3 adsorbeurs 130-132 sont mis en oeuvre dans l'installation, un adsorbeur est en phase de d'adsorption, pendant que les deux autres adsorbeurs sont en phase de régénération.

Le nombre de cartouches est dicté par le ratio temporel liant le temps (i.e. durée) requis pour adsorber un volume donné de CO₂ et le temps requis pour désorber ce même volume, c'est-à-dire en phase de régénération. Ici, ce ratio temporel est de 2, à savoir exemple 30 min pour l'adsorption et 60 min pour la désorption. Le nombre de cartouches, i.e. d'adsorbeurs 130-132, nécessaires est alors égal à ce ratio plus 1, c'est-à-dire trois cartouches.

Dans le cas d'utilisation d'autres adsorbants, par exemple des MOF, une plus grande capacité d'adsorption pourrait être trouvée, ce qui permettrait de gagner en compacité, donc utiliser moins d'adsorbant, mais d'augmenter le ratio temporel du fait d'affinité accrue entre les MOF et les molécules de CO₂ et de vapeur d'eau, par exemple, ce ratio pourrait être porté à 3, soit 60 min pour l'adsorption et 180 min pour la désorption, avec mise en oeuvre de 4 cartouches d'adsorption.

Pour gagner en compacité, en conjonction ou non d'un adsorbant à plus grande capacité d'adsorption, tel que des MOF ou autres, on peut aussi intégrer en amont des cartouches, en particulier dans la portion du conduit d'entrée 100 située en aval du premier capteur de débit 101, et donc en amont de la première vanne rotative 103, un (ou des) compresseur permettant d'augmenter la pression dans le ou les conduits, notamment les premier conduit 120, second conduit 121 et/ou troisième conduit 122, en communication fluidique avec ledit conduit d'entrée 100, selon la configuration de la première vanne rotative 103.

Cette augmentation de pression se transmet dans la cartouche correspondante, i.e. l'adsorbeur, pour augmenter la pression partielle du CO₂ par exemple, ce qui permet d'augmenter la capacité d'adsorption de l'adsorbant et donc de capturer une plus grande quantité de CO₂, et/ou bien de réduire la taille de la cartouche pour une quantité adsorbée équivalente, c'est-à-dire sans compression.

L'installation de fourniture de gaz respiratoire de l'invention est adaptée à l'administration de gaz à un patient en état hypoxémique, notamment une personne en état hypoxémique infectée par un coronavirus, telle Covid-19 ou analogue, que ce soit un adulte, notamment une personne âgée, un adolescent ou enfant.

## Revendications

1. Installation de fourniture (50) d'un gaz respiratoire à un utilisateur (P) comprenant :
- une source de gaz (3) pour fournir un gaz respiratoire contenant de l'oxygène,
- une interface respiratoire (21) pour administrer le gaz respiratoire à l'utilisateur, lors de chaque phase inspiratoire dudit utilisateur (P),
- une ligne principale d'acheminement de gaz (23) reliant fluidiquement la source de gaz (3) à l'interface respiratoire (21) pour acheminer le gaz respiratoire de la source de gaz (3) à l'interface respiratoire (21),
- une ligne de récupération de gaz (10) en communication fluidique avec l'interface respiratoire (21) pour récupérer et acheminer au moins une partie du mélange gazeux CO₂/O₂ expiré se retrouvant dans l'interface respiratoire (21), lors de chaque phase expiratoire de l'utilisateur (P),
- un système de purification de gaz (1) alimenté en mélange gazeux CO₂/O₂ expiré par la ligne de récupération de gaz (10), et configuré pour :
▪ éliminer au moins une partie du CO₂ contenu dans le mélange gazeux CO₂/O₂ expiré et
▪ obtenir un gaz purifié contenant majoritairement de l'O₂,
- et une ligne de recyclage de gaz (11) reliant fluidiquement le système de purification de gaz (1) à la ligne principale d'acheminement de gaz (23) pour acheminer au moins une partie du gaz purifié provenant du système de purification de gaz (1) et le réinjecter dans la ligne principale d'acheminement de gaz (23),
et dans laquelle le système de purification de gaz (1) comprend au moins trois adsorbeurs (130-132) agencés en parallèle contenant chacun au moins un adsorbant, chaque adsorbeur (130-132) étant configuré pour fonctionner selon des cycles d'adsorption/désorption comprenant :
▪ une phase d'adsorption pendant laquelle au moins une partie du CO₂ contenu dans le mélange gazeux CO₂/O₂ expiré est adsorbé par l'adsorbant, et
▪ une phase de régénération pendant laquelle au moins une partie du CO₂ adsorbé par l'adsorbant est désorbé et évacué à l'atmosphère,
**caractérisée en ce que** l'installation (50) comprend en outre une turbine (112) commandée par des moyens de pilotage (150, 151) à microprocesseur, ladite turbine (112) étant agencée sur un conduit d'air (110) relié fluidiquement à l'atmosphère et permettant de générer un débit d'air ambiant non-chauffé dans ledit conduit d'air (110), ledit débit d'air ambiant non-chauffé étant utilisé durant chaque phase de régénération pour régénérer au moins un adsorbant contenu dans au moins l'un des adsorbeurs (130-132) en désorbant au moins une partie du CO₂ adsorbé par ledit adsorbant.

2. Installation selon la revendication 1, **caractérisée en ce qu'**elle comprend trois adsorbeurs (130-132) agencés en parallèle, l'un des adsorbeurs (130-132) étant en phase d'adsorption et les deux autres adsorbeurs (130-132) étant en phase de régénération.

3. Installation selon la revendication 1, **caractérisée en ce que** la ligne de recyclage de gaz (11) comprend un réservoir-tampon (24) en communication fluidique avec la ligne principale d'acheminement de gaz (23), de préférence la ligne de recyclage de gaz (11) est raccordée fluidiquement au réservoir-tampon (24).

4. Installation selon la revendication 1, **caractérisée en ce que** l'interface respiratoire (21) est un masque respiratoire comprenant un orifice d'entrée d'oxygène (22) et un orifice de sortie de gaz expiré (25), la ligne principale d'acheminement de gaz (23) étant raccordée fluidiquement à l'orifice d'entrée d'oxygène (22) et la ligne de récupération de gaz (10) étant raccordée fluidiquement à l'orifice de sortie de gaz expiré (25).

5. Installation selon la revendication 1, **caractérisée en ce que** ledit au moins un adsorbant permet d'éliminer le CO₂ et la vapeur d'eau (H₂O).

6. Installation selon la revendication 1, **caractérisée en ce que** ledit au moins un adsorbant présentant une sélectivité supérieure pour le CO₂ que pour l'O₂ de manière à adsorber au moins une partie du CO₂ contenu dans le mélange gazeux CO₂/O₂ expiré.

7. Installation selon la revendication 1, **caractérisée en ce que** le système de purification de gaz (1) comprend une ou plusieurs vannes de distribution de gaz (103, 105) agencées en amont et/ou en aval des adsorbeurs (130-132) pour contrôler les entrées et sorties de gaz desdits adsorbeurs (130-132).

8. Installation selon la revendication 7, **caractérisée en ce que** les vannes de distribution de gaz (103, 105) comprennent une première vanne rotative (103) agencée en amont des adsorbeurs (130-132) et une deuxième vanne rotative (105) agencée en aval des adsorbeurs (130-132).

9. Installation selon les revendications 2 et 8, **caractérisée en ce que** la première et/ou la deuxième vanne rotative (103, 105) comprennent un raccord coudé mobile en rotation dans ladite première et/ou deuxième vanne rotative (103, 105) couplé mécaniquement à un actionneur rotatif piloté par les moyens de pilotage (150, 151) à microprocesseur.

10. Installation selon les revendications 1 et 2, **caractérisée en ce que** le système de purification de gaz (1) comprend en outre un premier capteur de débit (101) agencé en amont des adsorbeurs (130-132) et un second capteur de débit (102) agencé en aval des adsorbeurs (130-132), le premier et/ou le second capteur de débit (101, 102) coopérant avec les moyens de pilotage (150, 151).

11. Installation selon la revendication 10, **caractérisée en ce que** le conduit d'air (110) est relié fluidiquement à l'atmosphère par un orifice d'entrée d'air (110a), la turbine (112) étant agencée entre l'orifice d'entrée d'air (110a) et le second capteur de débit (102).

12. Installation selon la revendication 1, **caractérisée en ce que** les moyens de pilotage (150, 151) sont configurés pour déterminer une consigne de débit (Q) dans le conduit d'air (110).

13. Installation selon la revendication 12, **caractérisée en ce que** les moyens de pilotage (150, 151) sont configurés pour commander la turbine (112) pour délivrer un flux d'air dont le débit est égal ou approximativement égal à la consigne de débit (Q).

14. Installation selon la revendication 1, **caractérisée en ce que** les moyens de pilotage (150, 151) comprennent un microcontrôleur.

15. Installation selon la revendication 1, **caractérisée en ce que** la source de gaz (3) contient de l'oxygène gazeux.

## Patentansprüche

1. Anlage zur Abgabe (50) eines Atemgases an einen Benutzer (P), welche umfasst:
- eine Gasquelle (3) zur Abgabe eines Sauerstoff enthaltenden Atemgases,
- eine Atemschnittstelle (21) zur Verabreichung des Atemgases an den Benutzer während jeder Einatemphase des Benutzers (P),
- eine Gaszuführungshauptleitung (23), welche die Gasquelle (3) mit der Atemschnittstelle (21) fluidisch verbindet, um das Atemgas von der Gasquelle (3) zur Atemschnittstelle (21) zu befördern,
- eine Gasrückgewinnungsleitung (10), die mit der Atemschnittstelle (21) in Fluidverbindung steht, um wenigstens einen Teil des ausgeatmeten CO₂/O₂-Gasgemisches, das in der Atemschnittstelle (21) anfällt, während jeder Ausatemphase des Benutzers (P) rückzugewinnen und zu befördern,
- ein Gasreinigungssystem (1), das über die Gasrückgewinnungsleitung (10) mit ausgeatmetem CO₂/O₂-Gasgemisch gespeist wird und dafür ausgelegt ist:
▪ wenigstens einen Teil des in dem ausgeatmeten CO₂/O₂-Gasgemisch enthaltenen CO₂ zu beseitigen und
▪ ein gereinigtes Gas zu erhalten, das hauptsächlich O₂ enthält,
- und eine Gasrückführleitung (11), die das Gasreinigungssystem (1) mit der Gaszuführungshauptleitung (23) fluidisch verbindet, um wenigstens einen Teil des von dem Gasreinigungssystem (1) kommenden gereinigten Gases zu befördern und es in die Gaszuführungshauptleitung (23) rückzuführen,
und wobei das Gasreinigungssystem (1) mindestens drei parallel angeordnete Adsorber (130-132) umfasst, die jeweils mindestens ein Adsorptionsmittel enthalten, wobei jeder Adsorber (130-132) für einen Betrieb in Adsorptions-Desorptions-Zyklen ausgelegt ist, welche umfassen:
▪ eine Adsorptionsphase, während der wenigstens ein Teil des in dem ausgeatmeten CO₂/O₂-Gasgemisch enthaltenen CO₂ von dem Adsorptionsmittel adsorbiert wird, und
▪ eine Regenerationsphase, während der wenigstens ein Teil des von dem Adsorptionsmittel adsorbierten CO₂ desorbiert und an die Atmosphäre abgegeben wird,
**dadurch gekennzeichnet, dass** die Anlage (50) außerdem eine Turbine (112) umfasst, die von Ansteuermitteln (150, 151) mit einem Mikroprozessor gesteuert wird, wobei die Turbine (112) an einer Luftleitung (110) angeordnet ist, die mit der Atmosphäre fluidisch verbunden ist, und ermöglicht, einen Durchfluss von nicht erwärmter Umgebungsluft in der Luftleitung (110) zu erzeugen, wobei der Durchfluss von nicht erwärmter Umgebungsluft während jeder Regenerationsphase verwendet wird, um mindestens ein Adsorptionsmittel, das in mindestens einem der Adsorber (130-132) enthalten ist, zu regenerieren, indem wenigstens ein Teil des von diesem Adsorptionsmittel adsorbierten CO₂ desorbiert wird.

2. Anlage nach Anspruch 1, **dadurch gekennzeichnet, dass** sie drei parallel angeordnete Adsorber (130-132) umfasst, wobei einer der Adsorber (130-132) sich in einer Adsorptionsphase befindet und die zwei anderen Adsorber (130-132) sich in einer Regenerationsphase befinden.

3. Anlage nach Anspruch 1, **dadurch gekennzeichnet, dass** die Gasrückführleitung (11) einen Pufferbehälter (24) umfasst, der mit der Gaszuführungshauptleitung (23) in Fluidverbindung steht, und die Gasrückführleitung (11) vorzugsweise an den Pufferbehälter (24) fluidisch angeschlossen ist.

4. Anlage nach Anspruch 1, **dadurch gekennzeichnet, dass** die Atemschnittstelle (21) eine Atemmaske ist, die eine Sauerstoffeintrittsöffnung (22) und eine Austrittsöffnung für ausgeatmetes Gas (25) umfasst, wobei die Gaszuführungshauptleitung (23) an die Sauerstoffeintrittsöffnung (22) fluidisch angeschlossen ist und die Gasrückgewinnungsleitung (10) an die Austrittsöffnung für ausgeatmetes Gas (25) fluidisch angeschlossen ist.

5. Anlage nach Anspruch 1, **dadurch gekennzeichnet, dass** das mindestens eine Adsorptionsmittel ermöglicht, das CO₂ und den Wasserdampf (H₂O) zu beseitigen.

6. Anlage nach Anspruch 1, **dadurch gekennzeichnet, dass** das mindestens eine Adsorptionsmittel eine höhere Selektivität für CO₂ als für O₂ aufweist, so dass es wenigstens einen Teil des in dem ausgeatmeten CO₂/O₂-Gasgemisch enthaltenen CO₂ adsorbiert.

7. Anlage nach Anspruch 1, **dadurch gekennzeichnet, dass** das Gasreinigungssystem (1) ein oder mehrere Gasverteilungsventile (103, 105) umfasst, die stromaufwärts und/oder stromabwärts der Adsorber (130-132) angeordnet sind, um die Gaseinlässe und -auslässe der Adsorber (130-132) zu steuern.

8. Anlage nach Anspruch 7, **dadurch gekennzeichnet, dass** die Gasverteilungsventile (103, 105) einen ersten Drehschieber (103), der stromaufwärts der Adsorber (130-132) angeordnet ist, und einen zweiten Drehschieber (105), der stromabwärts der Adsorber (130-132) angeordnet ist, umfassen.

9. Anlage nach den Ansprüchen 2 und 8, **dadurch gekennzeichnet, dass** der erste und/oder der zweite Drehschieber (103, 105) ein im ersten und/oder zweiten Drehschieber (103, 105) drehbewegliches Kniestück umfassen, das mit einem Drehstellantrieb mechanisch gekoppelt ist, der von den Ansteuermitteln (150, 151) mit Mikroprozessor angesteuert wird.

10. Anlage nach den Ansprüchen 1 und 2, **dadurch gekennzeichnet, dass** das Gasreinigungssystem (1) außerdem einen ersten Durchflusssensor (101), der stromaufwärts der Adsorber (130-132) angeordnet ist, und einen zweiten Durchflusssensor (102), der stromabwärts der Adsorber (130-132) angeordnet ist, umfasst, wobei der erste und/oder der zweite Durchflusssensor (101, 102) mit den Ansteuermitteln (150, 151) zusammenwirken.

11. Anlage nach Anspruch 10, **dadurch gekennzeichnet, dass** die Luftleitung (110) mit der Atmosphäre durch eine Lufteinlassöffnung (110a) fluidisch verbunden ist, wobei die Turbine (112) zwischen der Lufteinlassöffnung (110a) und dem zweiten Durchflusssensor (102) angeordnet ist.

12. Anlage nach Anspruch 1, **dadurch gekennzeichnet, dass** die Ansteuermittel (150, 151) dafür ausgelegt sind, einen Durchflusssollwert (Q) in der Luftleitung (110) zu bestimmen.

13. Anlage nach Anspruch 12, **dadurch gekennzeichnet, dass** die Ansteuermittel (150, 151) dafür ausgelegt sind, die Turbine (112) so zu steuern, dass sie einen Luftstrom liefert, dessen Durchfluss gleich oder annähernd gleich dem Durchflusssollwert (Q) ist.

14. Anlage nach Anspruch 1, **dadurch gekennzeichnet, dass** die Ansteuermittel (150, 151) einen Mikrocontroller umfassen.

15. Anlage nach Anspruch 1, **dadurch gekennzeichnet, dass** die Gasquelle (3) gasförmigen Sauerstoff enthält.

## Claims

1. Installation (50) for supplying a respiratory gas to a user (P), comprising:
- a gas source (3) for supplying an oxygen-containing respiratory gas,
- a respiratory interface (21) for administering the respiratory gas to the user, during each inspiratory phase of said user (P),
- a main gas conveying line (23) fluidically connecting the gas source (3) to the respiratory interface (21) in order to convey the respiratory gas from the gas source (3) to the respiratory interface (21),
- a gas recovery line (10) in fluidic communication with the respiratory interface (21) in order to recover and convey at least some of the exhaled CO₂/O₂ gas mixture found in the respiratory interface (21), during each expiratory phase of the user (P),
- a gas purification system (1) supplied with exhaled CO₂/O₂ gas mixture via the gas recovery line (10), and configured to:
▪ eliminate at least some of the CO₂ contained in the exhaled CO₂/O₂ gas mixture and
▪ obtain a purified gas containing mainly O₂,
- and a gas recycling line (11) fluidically connecting the gas purification system (1) to the main gas conveying line (23) in order to convey at least some of the purified gas coming from the gas purification system (1) and to reinject it into the main gas conveying line (23),
and in which the gas purification system (1) comprises at least three adsorbers (130-132) arranged in parallel and each containing at least one adsorbent, each adsorber (130-132) being configured to operate according to adsorption/ desorption cycles comprising:
▪ an adsorption phase during which at least some of the CO₂ contained in the exhaled CO₂/O₂ gas mixture is adsorbed by the adsorbent, and
▪ a regeneration phase during which at least some of the CO₂adsorbed by the adsorbent is desorbed and evacuated to the atmosphere,
**characterized in that** the installation (50) further comprises a turbine (112) controlled by microprocessor-based control means (150, 151), said turbine (112) being arranged on an air duct (110) fluidically connected to the atmosphere and making it possible to generate a flow of unheated ambient air in said air duct (110), said flow of unheated ambient air being used during each regeneration phase to regenerate at least one adsorbent contained in at least one of the adsorbers (130-132) by desorption of at least some of the CO₂ adsorbed by said adsorbent.

2. Installation according to Claim 1, **characterized in that** it comprises three adsorbers (130-132) arranged in parallel, one of the adsorbers (130-132) being in the adsorption phase and the other two adsorbers (130-132) being in the regeneration phase.

3. Installation according to Claim 1, **characterized in that** the gas recycling line (11) comprises a buffer tank (24) in fluidic communication with the main gas conveying line (23), the gas recycling line (11) preferably being fluidically connected to the buffer tank (24).

4. Installation according to Claim 1, **characterized in that** the respiratory interface (21) is a breathing mask comprising an oxygen inlet orifice (22) and an exhaled gas outlet orifice (25), the main gas conveying line (23) being fluidically connected to the oxygen inlet orifice (22) and the gas recovery line (10) being fluidically connected to the exhaled gas outlet orifice (25).

5. Installation according to Claim 1, **characterized in that** said at least one adsorbent makes it possible to eliminate CO₂ and water vapour (H₂O).

6. Installation according to Claim 1, **characterized in that** said at least one adsorbent has a higher selectivity for CO₂ than for O₂, so as to adsorb at least some of the CO₂ contained in the exhaled CO₂/O₂ gas mixture.

7. Installation according to Claim 1, **characterized in that** the gas purification system (1) comprises one or more gas distribution valves (103, 105) arranged upstream and/or downstream of the adsorbers (130-132) in order to control the gas inlets and outlets of said adsorbers (130-132).

8. Installation according to Claim 7, **characterized in that** the gas distribution valves (103, 105) comprise a first rotary valve (103) arranged upstream of the adsorbers (130-132) and a second rotary valve (105) arranged downstream of the adsorbers (130-132).

9. Installation according to Claims 2 and 8, **characterized in that** the first and/or the second rotary valve (103, 105) comprise an elbow connector movable in rotation in said first and/or second rotary valve (103, 105) and mechanically coupled to a rotary actuator controlled by the microprocessor-based control means (150, 151).

10. Installation according to Claims 1 and 2, **characterized in that** the gas purification system (1) further comprises a first flow sensor (101) arranged upstream of the adsorbers (130-132) and a second flow sensor (102) arranged downstream of the adsorbers (130-132), the first and/or the second flow sensor (101, 102) cooperating with the control means (150, 151).

11. Installation according to Claim 10, **characterized in that** the air duct (110) is fluidically connected to the atmosphere by an air inlet orifice (110a), the turbine (112) being arranged between the air inlet orifice (110a) and the second flow sensor (102).

12. Installation according to Claim 1, **characterized in that** the control means (150, 151) are configured to determine a flow rate setpoint (Q) in the air duct (110).

13. Installation according to Claim 12, **characterized in that** the control means (150, 151) are configured to control the turbine (112) to deliver a flow of air whose flow rate is equal or approximately equal to the flow rate setpoint (Q).

14. Installation according to Claim 1, **characterized in that** the control means (150, 151) comprise a microcontroller.

15. Installation according to Claim 1, **characterized in that** the gas source (3) contains gaseous oxygen.
